# EUROPEAN PATENT SPECIFICATION

(11) **EP 1 451 302 B1**
(45) Date of publication and mention of the grant of the patent: **28.05.2008**
(21) Application number: 01274401.7
(22) Date of filing: 28.11.2001
(51) Int. Cl.: C12N 5/06, C12N 5/08, A61K 35/44

(54) **IN VITRO RECONSTITUTED SHEETS OF HUMAN CORNEAL EPITHELIUM AND METHOD OF PRODUCING THE SAME**
IN VITRO REKONSTITUIERTE LAGEN DES MENSCHLICHEN HORNHAUTEPITHELS UND VERFAHREN ZUR HERSTELLUNG DAVON
FEUILLETS DE L'EPITHELIUM CORNEEN HUMAIN RECONSTITUES IN VITRO ET METHODE DE PRODUCTION DE CES DERNIERS

(30) Priority: 03.08.2001 IT RM20010476
(43) Date of publication of application: 01.09.2004
(73) Proprietor: IDI FARMACEUTICI S.P.A., 00040 Pomezia RM (IT)
(72) Inventor: DE LUCA, Michele, 00040 Ardea, Roma (IT); PELLEGRINI, Graziella, 00040 Ardea, Roma (IT)
(74) Representative: Capasso, Olga
(86) International application number: PCT/IT2001/000603
(87) International publication number: WO 2003/012083

(56) References cited:
- EP-A- 0 572 364
- WO-A-96/13974
- WO-A-99/37752
- RAMA PAOLO ET AL: "Autologous fibrin-cultured limbal stem cells permanently restore the corneal surface of patients with total limbal stem cell deficiency." TRANSPLANTATION (BALTIMORE), vol. 72, no. 9, 15 November 2001 (2001-11-15), pages 1478-1485, XP001061855 ISSN: 0041-1337
- PELLEGRINI GRAZIELLA ET AL: "p63 identifies keratinocyte stem cells." PROCEEDINGS OF THE NATIONAL ACADEMY OF SCIENCES OF THE UNITED STATES, vol. 98, no. 6, 13 March 2001 (2001-03-13), pages 3156-3161, XP002192317 March 13, 2001 ISSN: 0027-8424
- PELLEGRINI , G. ET AL.: "Location and Clonal Analysis of Stem Cells and their Differentiated Progeny in the Human Ocular Surface" THE JOURNAL OF CELL BIOLOGY, vol. 145, no. 4, - 17 May 1999 (1999-05-17) pages 769-782, XP002192318

## Description

The present invention relates to oculistic implantation and more particularly to the in vitro production of sheets of human corneal epithelium containing stem cells from cultured limbar stem cells to be grafted directly to patients.

As known, in the corneal epithelium stem cells are confined in the layer between cornea and conjunctiva, so-called limbus, and allow the upper layers to be continuously renewed.

In severe ocular burns, the loss of corneal tissue and the total or subtotal depletion of limbar cells induces the missing epithelium to be replaced by conjunctival cells, which naturally form an opaque layer causing the loss of sight.

Grafts of tissue sheets containing limbar cells allows the functionality of cornea to be recovered, the blindness due to the destructive lesion of the limbar-corneal epithelium to be overcome, and the perforating keratoplasty to be successfully carried out, even in the most complex cases which show an extremely small number of successful results using the known methods.

It is well known that epithelial cells are so difficult to be cultured and maintained in vitro as they are fragile and difficult to handle that hitherto used methods to produce tissues in vitro are little effective.

The present invention seeks to effectively select the limbar cells to be cultivated in vitro and to improve the method of cultivating the same on a fibrin substrate suitably modified to obtain reconstituted sheets of corneal epithelium ready to be used in grafts. More particularly, the in vitro reconstitution protocol has been improved in order to guarantee reproducibility and extreme simplicity of use of the reconstituted corneae provided by such protocol.

The human anterior ocular surface is covered with two highly specialised structures: conjunctive and cornea. Cornea refracts light and directs it to the visual perception region of retina being responsible for focusing the images along with crystalline. The conjunctive covers the remaining bulbi oculi portion and performs an important function in keeping the normal homeostasis of the ocular surface. Although the conjunctival and limbar-corneal tissues have the typical characteristics of the epithelia, they are formed by two genotipically different cell types, hereafter referred to as conjunctival keratinocytes and limbar-corneal keratinocytes, respectively. In particular, corneal epithelium is renewed constantly and continuously because of the stem cells confined in the deepest transitional layer between cornea and conjunctive, so-called limbus, where the transient amplifying cells (TA cells) forming the corneal epithelium are originated. Corneal epithelium is completely renewed every 9-12 months.

The *in vitro* reconstitution of epithelial tissues aiming at repairing and/or replacing the natural ones, for example, as a result of severe burns, is widely supported by literature. Cells are cultivated in vitro on suitable biological substrates helping the cell adherence and proliferation as well as allowing the proper architecture of the epithelial tissue to be obtained. One of the most used substrate is fibrin which is available even in commercial forms as TISSUCOL (by Baxter-Immuno, Vienna, Austria), as a solution of two components: fibrinogen and thrombin. Such composition prepared according to the protocol provided by the manufacturer, however, is not suitable to be used in cultures of limbar-corneal keratinocytes because it does not have the proper transparency and elasticity.

Normally used *in vitro* cell cultivation protocols provide that the propagation occurs within flasks, wells, Petri dishes, or other sterile containers of glass or plastics such as polystyrene. As these surfaces do not show any sufficient adhesiveness to the cells, the containers on the market are pretreated chemically or irradiated such as to increase the positive charges on their surfaces to interact with the negatively charged cell membranes and to help cell adhesion.

The present invention overcomes the problems related to the preparation of homogeneous, steady cultivated corneal stem cells. Said cells growing on a suitably modified fibrin substrate originate in vitro sheets of corneal epithelium having all of the characteristics of the human cornea such as size, thickness and transparency. In addition, this method allows the elastic sheets of corneal epithelium to adhere perfectly to the ocular surface without having to cause it to bend during the development in vitro (as described by the same inventor in his Italian Patent No. 1255657). Finally, the preparation of said sheets of human corneal epithelium on a modified fibrin substrate is compulsorily executed on dishes and/or containers not-treated for cell cultures to provide a final product having the desired characteristics of thickness, density and transferability. Therefore, the sheets of human corneal epithelium produced in vitro according to the present invention are ready to be used and adapted to any patient. The present invention refers to a method whereby it is possible to prepare in laboratory sheets of human corneal epithelium to be used in grafts. Such method requires a number of steps to obtain homogeneous cell cultures containing stem cells which are capable of easily forming in vitro sheets of human corneal epithelium ready to use thanks to the modified fibrin substrate.

It is an object of the invention a method for *in vitro* producing sheets of human corneal epithelium, characterized by the following steps:
a) identifying limbar stem cells of a biopsy of the limbar ocular region using limbar stem cell specific markers,
b) selecting said identified limbar stem cells by clonal analysis;
c) confirming the selection of limbar stem cells by using limbar stem cell specific markers;
d) growing said selected limbar stem cells on a fibrin substrate obtained by solubilizing 2 to 5 OIU/ml thrombin and fibrinogen in a sodium and calcium chloride solution having a sodium concentration between 0.001 and 0.5 millimoles/0.6 ml and a chloride concentration between 0.003 and 0.2 millimoles/0.6 ml, said substrate being dispensed in a non-treated solid support, such that cells do not interact with the surface of said support.

Preferably the specific markers are included in the group of keratin 19, keratin 12, keratin 3 and protein p63.

Preferably the sheets of human corneal epithelium have characteristics of size, density, transparency, elasticity, thickness such that they can be immediately grafted and the modified fibrin substrate resorpted after graft.

Preferably the modified fibrin substrate has a thickness between 50 and 300 micron, more preferably of 100 micron. In a preferred aspect the non treated solid support is a ring, more preferably the ring has a size compatible with the human cornea, most preferably having a diameter between 15 and 40 mm and a height between 3 and 8 mm.

In a specific embodiment the diameter is 33 mm.

Further features and advantages of the invention will be more readily apparent from the following detailed description of an embodiment with reference to the accompanying drawings. In the drawings:
Figure 1 shows an histologic section of a layer of cells taken from the limbar region, fixed and coloured with specific antibodies K3 and K19.
Figure 2 shows an histologic section of a layer of conjunctival cells fixed and coloured with specific antibodies K3 and K19.
Figure 3 shows clones obtained by bioptic samples of the limbar region.
Figure 4 shows the morphological aspect of a holoclone.
Figure 5 shows sheets of corneal epithelium obtained in vitro.

### PREPARATION OF CELL CULTURES

As described above, the cells responsible for the continuous renewal of the corneal epithelium are the cells of the limbar layer. The limbar layer also contain stem cells and for this reason it is capable of regenerating all over again the whole epithelium as such. Object of the invention is a new protocol for the in vitro preparation of cultured limbar stem cells which comprises the following steps:

### 1. Selection of cells taken from the limbus.

Cell samples are taken from the limbar ocular region by biopsy. As mentioned above, the limbus is the transient layer between conjunctival epithelium and corneal epithelium. For this reason, in order to provide cell cultures mainly formed of corneal cells it is necessary to clearly distinguish between the two cell types. Specific markers allow the corneal cells to be distinguished genotipically from the underlying conjunctival cells. It is well known that the conjunctival epithelium produces cytokeratin 19, while the corneal epithelium produces specifically cytokeratin 3 and 12.

Therefore, cell cultures from biopsy material are detected using an immunotest with specific antibodies for cytokeratin 3 and cytokeratin 19 by a known technique.

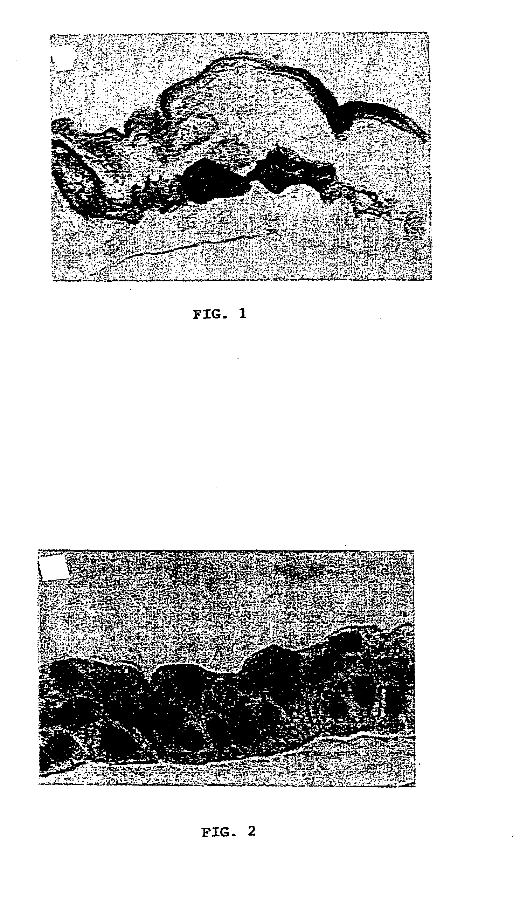

Figures 1 and 2 show how different cell preparations react to specific antibodies. In particular, Figure 1 shows the corneal cells expressing the K3+/K19-phenotype, while Figure 2 shows the conjunctival cells with phenotype K3-/K19+. In this way only cell cultures containing conjunctival cells in a rate lower than 50% can be selected. As a result, the obtained cell cultures with corneal cells expressing the K3+ phenotype and negative for the specific marker K19, contain corneal stem cells.

Thus, after having provided cell preparations of corneal and limbar cells in a proportion greater than 50%, it is necessary to identify corneal stem cells.

### 2. Selection of limbar cells by clonal analysis.

As previously described, limbar stem cells originate transient amplifying cells (TA) which differentiate definitively after a number of cellular divisions.

The clonal analysis allows the stem cells to be selected in order to investigate their capability to be propagated in culture. Therefore, cells are classified as holoclones, meroclones, and paraclones by this technique.

Holoclones are formed by cells belonging to the basal limbus layer which show a high proliferative capacity (being able to undergo 100 divisions) and are capable to generate *in vivo* a mature epithelium and to differentiate into distinct cellular lineages. Holoclones are generated by stem cells of limbus [Pellegrini, G. et al., 1999, J. Cell Biol., 145, 769-782].

Paraclones are formed by TA cells that divide 15 times as a maximum and originate colonies formed by cells at the terminal stage of differentiation.

Meroclones are formed by young TA cells which have a proliferative capacity greater than paraclones. The conversion from holoclones to meroclones and paraclones is irreversible.

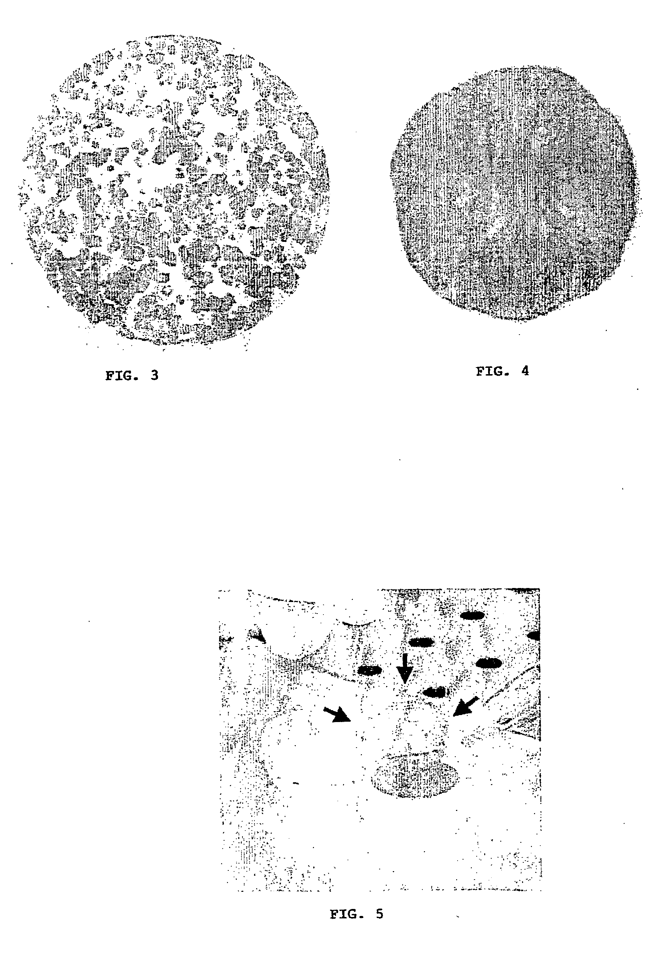

As shown in Figures 3 and 4, only cells from limbar region produce large, smooth colonies, the holoclones, and can be cultivated up to 14 passages (2-3 months) and undergo 80-100 cellular divisions before senescence.

### 3. Selection of limbar stem cells using specific markers for limbar cells.

According to the present invention, the results obtained with clonal analysis have to be confirmed univocally by further analysis using specific markers for limbar cells.

Protein p63, a transcription factor of the same family of p53 and p73 proteins, is used as a specific marker [Pellegrini, G. et al., 2001, Proc. Natl. Acad. Sci. USA, 98, 3156-3161]. As known, p53 plays an important role in tumours inhibition, while p63 and p73 are involved in cell differentiation.

The results have pointed out that p63 expression in the holoclone cells, i.e. stem cells, is 200 times higher than in the paraclone cells.

The specific expression of p63 in stem cells is also confirmed by comparing its expression with the expression of PCNA (nuclear antigen of the proliferating cells), which is a characteristic feature of active proliferating cells. The results show that p63 is expressed in stem cells, irrespectively of their proliferative state, but not in replicating cells.

As a result of the selection processes described above, cell cultures mainly consisting of stem cells and able to regenerate in vitro corneal tissue are obtained. Furthermore, it is shown that such corneal tissue is positively capable of keeping its physiologic functions for at least 5 years after being grafted in a patient.

### MODIFICATION OF THE FIBRIN SUBSTRATE

It is well-known that fibrin is the product of the polymerisation reaction of fibrinogen induced by thrombin. This substance is a sealant that help cells forming continuous and homogeneous layers.
The components are known under the commercial name TISSUCOL (Baxter-Immuno, Vienna, Austria). As already mentioned, the protocol of preparation of the commercial product is not suitable for *in vitro* reconstitution of sheets of human corneal epithelium because it is not transparent and elastic enough.

In order to overcome this problem, the preparation protocol has been suitably modified in order to obtain thrombin and fibrin solutions that, when mixed, provide a fibrin gel having the required characteristics of flexibility and transparency.

This is obtained by modifying the ionic strength of said solutions by adding salts such as sodium chloride and calcium chloride and consequently modulating the polymerisation reaction. Following this protocol, it is possible to have a final product with precise chemical and physical characteristics. In particular, the fibrin gel obtained by said process contains sodium in a concentration between 0.5 and 0.001 millimoles and chlorine in a concentration between 0.2 and 0.003 millimoles.

Both the solutions for the solubilisation of thrombin and for the solubilization of fibrin are then prepared by mixing NaCl, CaCl₂ and distilled H₂O.

The thrombin and fibrinogen packages of the commercial form TISSUCOL (Baxter-Immuno, Vienna, Austria) are defrosted in a thermostated bath at 37°C. Thereafter, an amount of the thrombin solution is transferred from the package to the test-tube and diluted by the previously prepared solution for the solubilisation of thrombin until a thrombin concentration between 2 and 5 OIU/ml is obtained. In the same way an amount of fibrin is transferred to a Petri dish and mixed with the previously prepared solution for the solubilisation of fibrin.

Now the solutions prepared by this process are dispensed, in sterile conditions, in a dish for bacteriology, where the circular shape is obtained by confining the fibrin substrate within a ring with a little larger diameter than that of the human cornea in order to provide sheets of human corneal epithelium able to exactly fit in with the ocular surface, and capable of maintaining the suitable size after being grafted and the consequent resorption of the fibrin layer substrate. The use of not-treated for cell cultures dishes and/or containers is essential to provide sheets of corneal epithelium having the specific characteristics of thickness, density, and transferability needed for surgery.

The ring is prepared by cutting the upper end of a 50 ml test-tube with a diameter between 20 and 40 mm at a height between 3 and 8.0 mm by a white-hot tungsten wire. The object obtained is then sterilised by autoclavation.

After gentle stirring to distribute the gel uniformly, the dish is kept, in sterile conditions, up to a complete polymerisation and stored at 4°C for a time from 1 hour up to 1 month.

The fibrin gel formed as a result of the polymerisation reaction has the desired chemical-physical features and is characterised in that it includes sodium in a concentration between 0.5 and 0.001 millimoles and chlorine in a concentration between 0.2 and 0.003 millimoles in the final gel amount of 0.6 ml.

The fibrin layer in the form of gel obtained by this process has a thickness between 50 and 300 microns and a diameter between 15 and 40 mm and is elastic and transparent.
*IN VITRO* RECONSTITUTION OF SHEETS OF CORNEAL EPITHELIUM Limbar keratinocytes cultures, obtained as previously described, are treated with trypsin and put on a dish inside the fibrin ring (cellular density between 2.5x10⁴ cells/cm² and 1x10⁵ cells/cm²) in the presence of a nutritive layer of lethal irradiated 3T3-J2 cells (murine embryonal fibroblasts) having the characteristics described above. At confluence, the epithelial layers obtained on the modified fibrin substrate are washed with Dulbecco Modified Eagle's Medium (DMEM) including glutamine, penicillin and streptomycin and stored into a case for contact lenses (Bausch and Lomb) in the presence of DMEM, as shown in Figure 5. Sheets of human limbar-corneal epithelium obtained by this process are ready to be used in therapy.

### EXAMPLE

At confluence, cultured cells obtained from biopsies are removed from culture flasks with Dispase II (Green, H., et al., 1979, Proc. Natl. Acad. Sci. USA, 76, 5665-5668), fixed in formaldehyde (4% in PBS) for 12 hours at 4°C and put into paraffin. The sections are coloured with haematoxylin-eosin or specific monoclonal antibodies for cytokeratin 3 (AE5 mAB; DAKO) and cytokeratin 19 (RCK108 mAB; DAKO). The same coloration protocol is also carried out within 24 hours on cell samples removed from a non-confluent culture, cytocentrifuged on a glass and dried.

The specific cytokeratin 3 antibody bounded to the substrate is identified by using the HRP-dextran-anti-mouse complex (*EnVision* Plus/HPR system DAKO) and 3,3'-diaminobenzidine tetrachlorate (FAST DAB; Sigma Chemical Co.) as chromogen.

The specific cytokeratin 19 antibody bound to substrate is identified by using the alkaline phosphatase-dextran-anti-mouse complex (EnVision Plus/HPR system DAKO) and Fast rED TR/Naphtol AS-MX (FAST Red; Sigma Chemical Co.) as chromogen.

The presence of corneal stem cells is detected by clonal analysis. Then single cells isolated by microscope are inoculated in wells containing a nutritive layer of 3T3-J2 cells (murine embryonal fibroblasts) (Rochat, A. et al., 1994, Cell, 76, 1063-1073) cultivated up to the tenth passage and amplified to obtain a maximum rate of 1:10. After 7 days the clones are identified by microscope, their size is measured, and they are transferred onto a dish containing a layer of 3T3-J2 cells. After 12 days the content of the dish is fixed by rhodamine B.

The clonal type is determined by measuring the proportion of abortive colonies (Barrandon, Y. and Green, H., 1987, Proc. Natl. Acad. Sci. USA, 84, 2302-2306).

The results obtained by clonal analysis are confirmed by the analysis conducted by using protein p63.

Cellular cultures, both from confluent biopsies and previously identified clones, are removed from culture flasks with Dispase II (Green, H., et al., 1979, Proc. Natl. Acad. Sci. USA, 76, 5665-5668), fixed in formaldehyde (4% in PBS) for 30 minutes at room temperature and put into paraffin.

The treated cells, or an amount thereof removed from a non-confluent culture, cytocentrifuged on a glass and fixed after 24 hours with a mixture of oxygen peroxide and methanol for 5 minutes at 20°C, are coloured with specific monoclonal antibodies for p63 (given by Frank Kckeon) and specific antibody for PCNA (nuclear antigen of the proliferating cells) (Santa Cruz Biotechnology) according to known techniques (Yang, A. et al., 1999, Nature, 398, 714-718; Parsa, R. et al., 1999, J. Invest. Dermatol., 113, 1099-1105; Bravo, R. et al., 1987, Nature, 326, 515-517). The cells are extracted for the blotting with a RIPA buffer (0.15 mM NaCl/0.05 mM Tris HC1, pH 7.2/1% Triton X-100/1% sodium deoxycholate/0.1% SDS), as already described by the same inventor (Dellambra, E. et. al., 2000, J. Cel. Biol., 149, 117-1129). Analogous samples are analysed with electrophoresis on a polyacrylamide/SDS gel and transferred to filters of polyvynilidenedifluoride (PVDF) (Immobilon-P, Millipore). The immunologic reactions are executed as described above and the antibodies bound to the filter are detected by chemiluminescence with ECL (Amersham Pharmacia).

As described above, cell cultures obtained by the above process are formed by 95% corneal stem cells.

The solution for the solubilization of thrombin is prepared by mixing 11 ml of 10% NaCl with 1 ml of 100 mM CaCl₂ in 88 ml distilled H₂O. Likewise the solution for the solubilization of fibrin is prepared by mixing 1038 microlitres of 10% NaCl, 1.74 ml of 0.9% NaCl, and 108 microlitres of 100 mM CaCl₂ in 2914 microlitres of distilled H₂O. The packages of thrombin and fibrinogen are kept in a thermostated bath at 37°C until they are completely defrosted. Thereafter, 0.5 ml of the thrombin solution is transferred from the package to a test-tube and diluted with 4.5 ml of the previously prepared solution for the solubilization of thrombin, whereupon 1.5 ml of such solution is further diluted with 25 ml of the solution for the solubilization of thrombin to obtain a thrombin concentration of 3 OIU/ml. In the same way 5 ml of fibrin is transferred onto a Petri dish and mixed with 5.8 ml of the previously prepared solution for the solubilization of fibrin.

Then, 0.3 ml each of the above solutions are distributed, in sterile conditions, in a dish for bacteriology, where the ring prepared by cutting the upper end of a 50 ml test-tube with a diameter of 30 mm at a height of 8.0 mm by a white-hot tungsten wire has been arranged, and then sterilised by autoclave.

After gentle stirring to uniformly distribute the gel, the dish is kept, in sterile conditions, up to a complete polymerisation and stored at 4°C for a time from 1 hour up to 1 month.

The fibrin gel formed as a result of the polymerisation reaction is characterised in that it includes sodium in a concentration between 0.08 and 0.16 millimoles and an optimum amount of chlorine in a concentration between 0.03 and 0.01 millimoles. The precise ratio between sodium and calcium chlorides gives to said gel the desired chemical-physical characteristics.

The fibrin gel layer obtained by this process has a thickness of 100 microns and a diameter of 33 mm and is elastic and transparent. Finally the sheets of corneal epithelium ready to be grafted are obtained by treating with trypsin the cultures of limbar keratinocytes prepared as described above with Dispase II and putting them on a dish on the fibrin ring with cell density between 2.5x10⁴ cells/cm² and 1x10⁵ cells/cm² in the presence of a nutritive layer of 3T3-J2 cells. At confluence, the epithelial layers on the fibrin substrate are washed by Dulbecco Modified Eagle's Medium (DMEM) including 4 mM glutamine, 50 IU/ml penicillin and 50 µg/ml streptomycin and stored into a case for contact lenses (Bausch and Lomb) in the presence of DMEM.

Sheets of human corneal epithelium reconstituted by this process have been clinically tested on 18 patients suffering from loss of sight due to severe burns caused by chemical agents, 11 of whom were affected by total loss of limbus and 3 patients were affected by severe depletions of the limbar region. Implantation of reconstituted corneae *in vitro* gave positive results in 14 out of 18 patients. More exactly, the corneal epithelium is completely recovered after about 7 days, all symptoms connected to the inflammatory and vascularization processes regress after 3-4 weeks, and the cornea surface looks covered by a smooth, transparent epithelium after one month. Finally, the cornea epithelium recovers completely its natural characteristics after 12-24 months and the sharpness of sight of the patients becomes normal again in case of non-severe damages, while such method allows the so-called perforating keratoplasty to be successfully executed and the sight to be later recovered in case of severe damages. In addition, such method avoids graft rejections due to transplants from heterologous donors in case the patient has retained 1 mm of limbus region at least in one of his eyes.

In conclusion, the present invention easily allows to obtain in vitro sheets of human corneal epithelium which can be used in grafts having size and transparency similar to the human ones. Furthermore, the fibrin substrate prepared by this method helps cell adhesion after the graft because it is promptly reabsorbed without giving any allergic reaction to the patient. In addition, the use of not treated for cell cultures dishes and/or containers of plastics or glass allows to obtain a final product having optimum characteristics of thickness, density, and transferability for its use in surgery. Finally, as mentioned above, said corneal tissue is able to maintain its physiologic functions for at least 5 years after being grafted in patients.

## Claims

1. A method for *in vitro* producing sheets of human corneal epithelium, **characterized by** the following steps:
a) identifying limbar stem cells of a biopsy of the limbar ocular region using limbar stem cell specific markers,
b) selecting said identified limbar stem cells by clonal analysis;
c) confirming the selection of limbar stem cells by using limbar stem cell specific markers;
d) growing said selected limbar stem cells on a fibrin substrate obtained by solubilizing 2 to 5 OIU/ml, thrombin and fibrinogen in a sodium and calcium chloride solution having a sodium concentration between 0.001 and 0.5 millimoles/0.6 ml and a chloride concentration between 0.003 and 0.2 millimoles/0.6 ml, said substrate being dispensed in a non-treated solid support, such that cells do not interact with the surface of said support.

2. The method according to claim 1 wherein said specific markers are included in the group of keratin 19, keratin 12, keratin 3 and protein p63.

3. The method according to claim 1 wherein said sheets of human corneal epithelium have characteristics of size, density, transparency, elasticity, thickness such that they can be immediately grafted and the modified fibrin substrate resorpted after graft.

4. The method according to any of previous claims wherein the modified fibrin substrate has a thickness between 50 and 300 micron.

5. The method according to claim 4 wherein the thickness is 100 micron.

6. The method according to claim 5 wherein the non treated solid support is a ring.

7. The method according to claim 6 wherein the ring has a size compatible with the human cornea.

8. The method according to claim 7 wherein the ring has a diameter between 15 and 40 mm and a height between 3 and 8 mm.

9. The method according to claim 8 wherein the diameter is 33 mm.

## Patentansprüche

1. Verfahren zur in vitro-Herstellung von Lagen aus menschlichem Hornhautepithel, **gekennzeichnet durch** die folgenden Schritte:
a) Identifizieren von Limbus-Stammzellen aus einer Biopsie des Limbus-Augenbereichs unter Verwendung von Limbus-Stammzellen-spezifischen Markern;
b) Selektieren der identifizierten Limbus-Stammzellen **durch** Klonanalyse;
c) Bestätigen der Selektion von Limbus-Stammzellen unter Verwendung von Limbus-Stammzellen-spezifischen Markern;
d) Züchten der selektierten Limbus-Stammzellen auf einem Fibrinsubstrat, das **durch** Solubilisieren von 2 bis 5 OIU Thrombin/ml und Fibrinogen in einer Natrium- und Calciumchloridlösung mit einer Natriumkonzentration zwischen 0,001 und 0,5 Millimol/0,6 ml und einer Chloridkonzentration zwischen 0,003 und 0,2 Millimol/0,6 ml erhalten wurde, wobei das Substrat in einen unbehandelten festen Träger abgegeben wird, so dass die Zellen mit der Oberfläche des Trägers nicht in Wechselwirkung treten.

2. Verfahren nach Anspruch 1, wobei die spezifischen Marker in die Gruppe von Keratin 19, Keratin 12, Keratin 3 und Protein p63 eingeschlossen sind.

3. Verfahren nach Anspruch 1, wobei die Lagen des menschlichen Hornhautepithels Charakteristika der Größe, Dichte, Transparenz, Elastizität, Dicke aufweisen, so dass sie unmittelbar transplantiert werden können und das modifizierte Fibrinsubstrat nach dem Transplantieren resorbiert werden kann.

4. Verfahren nach einem der vorhergehenden Ansprüche, wobei das modifizierte Fibrinsubstrat eine Dicke zwischen 50 und 300 µm hat.

5. Verfahren nach Anspruch 4, wobei die Dicke 100 µm beträgt.

6. Verfahren nach Anspruch 5, wobei der unbehandelte feste Träger ein Ring ist.

7. Verfahren nach Anspruch 6, wobei der Ring eine Größe hat, die mit der menschlichen Hornhaut kompatibel ist.

8. Verfahren nach Anspruch 7, wobei der Ring einen Durchmesser zwischen 15 und 40 mm und eine Höhe zwischen 3 und 8 mm hat.

9. Verfahren nach Anspruch 8, wobei der Durchmesser 33 mm beträgt.

## Revendications

1. Procédé de production *in vitro* de feuilles d'épithélium cornéen humain, **caractérisé par** les étapes suivantes :
a) identification des cellules souches limbaires d'une biopsie de la région oculaire limbaire en utilisant des marqueurs spécifiques pour les cellules souches limbaires ;
b) sélection desdites cellules souches limbaires identifiées par analyse clonale ;
c) confirmation de la sélection des cellules souches limbaires en utilisant des marqueurs spécifiques pour les cellules souches limbaires ;
d) culture desdites cellules souches limbaires sélectionnées sur un substrat de fibrine obtenu en solubilisant 2 à 5 OIU/ml de thrombine et du fibrogène dans une solution de chlorure de sodium et de calcium ayant une concentration en sodium comprise entre 0,001 et 0,5 millimole/0,6 ml et une concentration en chlorure comprise entre 0,003 et 0,2 millimole/0,6 ml, ledit substrat étant dispensé dans un support solide non traité, de sorte que les cellules n'interagissent pas avec la surface dudit support.

2. Procédé selon la revendication 1 dans lequel lesdits marqueurs spécifiques sont inclus dans le groupe de la kératine 19, de la kératine 12, de la kératine 3 et de la protéine p63.

3. Procédé selon la revendication 1 dans lequel lesdites feuilles d'épithélium cornéen humain ont des caractéristiques de taille, de densité, de transparence, d'élasticité et d'épaisseur telles qu'elles peuvent être immédiatement greffées et le substrat de fibrine modifiée subir une résorption après greffe.

4. Procédé selon l'une quelconque des revendications précédentes dans lequel le substrat de fibrine modifiée a une épaisseur comprise entre 50 et 300 microns.

5. Procédé selon la revendication 4 dans lequel l'épaisseur est de 100 microns.

6. Procédé selon la revendication 5 dans lequel le support solide non traité est un anneau.

7. Procédé selon la revendication 6 dans lequel l'anneau a une taille compatible avec la cornée humaine.

8. Procédé selon la revendication 7 dans lequel l'anneau a un diamètre compris entre 15 et 40 mm et une hauteur comprise entre 3 et 8 mm.

9. Procédé selon la revendication 8 dans lequel le diamètre est de 33 mm.
